# EUROPEAN PATENT SPECIFICATION

(11) **EP 4 200 189 B1**
(45) Date of publication and mention of the grant of the patent: **28.01.2026**
(21) Application number: 21857100.8
(22) Date of filing: 19.10.2021
(51) Int. Cl.: B62B 5/06, A61L 2/238, C09J 7/29

(54) **RETROFIT SANITARY HANDLE**
NACHRÜSTBARER SANITÄRGRIFF
POIGNÉE À ADAPTATION SANITAIRE

(43) Date of publication of application: 28.06.2023
(73) Proprietor: Dajcor Aluminum Ltd., Chatham, ON N7M 0N5 (CA)
(72) Inventor: LOUCKS, William J., Chatham, Ontario N7L 3A6 (CA)
(74) Representative: Definition IP Limited
(86) International application number: PCT/CA2021/051472
(87) International publication number: WO 2022/036465

(56) References cited:
- WO-A1-97/15039
- DE-A1- 102011 050 881
- DE-A1- 102019 102 970
- DE-U1- 202008 010 201
- DE-U1- 202013 001 211
- DE-U1- 202020 102 495
- DE-U1- 202020 102 495
- FR-B1- 2 947 509
- US-A1- 2007 267 828
- US-A1- 2009 202 845
- US-A1- 2012 148 783
- US-A1- 2017 013 969
- US-A1- 2020 398 883
- US-B1- 6 854 163
- US-B2- 10 166 158
- US-B2- 8 900 716
- US-B2- 9 127 189

## Description

### Cross Reference to Prior Applications

For the purposes of the United States of America, this application is a Continuation In Part of U.S. Patent Application No. 16/873,941 , filed on August 20, 2020. This application claims priority to U.S. Patent Application No. 16/873,941 , filed on August 20, 2020.

### Background

There are many surfaces that people touch in normal life that have the possibility of transmitting disease or illness-causing pathogens. For examples microbes (bacteria, protozoa, fungi, algae, amoebas, and slime molds), viruses, and like harmful organisms/pathogens can exist on handles, rails, and other surfaces for significant periods of time and can be transferred onto a human's skin when touched. If the human then touches her/his face, or the pathogen otherwise come into contact with sensitive tissue on the human's body, illness or disease may ensue. Common surfaces with this issue include shopping cart handles, hospital bed railings, fuel pump handles, commercial building door handles, and staircase railings.

There have been many proposals for dealing with the problem of pathogen transfer from a handle or railing to a human user. For example, USP 6,817,066 provides a removable foam rubber grip for a shopping cart handle that prevents the shopper's hands from coming into contact with a conventional grocery cart handle. USPs 5,215,319 and 5,820,142 provide removable rigid plastic sanitary coverings for a shopping cart handle, and USP 8,109,524 utilizes an elongated flexible shopping cart handle cover with an interior anti-bacterial composition. USP 10,166,158 shows a rail cover assembly having top and bottom components made of antimicrobial or biocidal materials that couple together around railings to provide a sanitary gripping surface, and US Patent Publication No. 2005/0267233 teaches using a wide variety of antimicrobial components on a handle to allow sanitary usage of the handle. While all of these proposals can be effective under some circumstances, the present description seeks to provide a system and method that are more robust and permanent than are provided by most of the above teachings for preventing or at least minimizing the chances of pathogens from transferring from a handle or rail to a human's skin.

DE202020102495 describes a protective device for protecting the operator of a device, the protective device being detachably fixed to the handle of the device and having a support surface for supporting the device on the handle, a gripping surface to be held by the operator, an insulating section for isolating the gripping surface from the handle and a recess for receiving the handle.

US2009/202845 describes a sheet of anodized aluminium which is heat treated and coated with an antimicrobial composition.

The antimicrobial coating is bound to the surface of the anodic layer and comprises a network of cross-linked organo-silane molecules that are covalently bonded to the surface of the anodic layer.

US2017/013969 describes a rail cover assembly comprising a first cover portion and a second cover portion, each having a surface made from an anti-microbial material. A spring-loaded joiner is engaged with each of the cover portions.

### Summary

According to the invention, the disadvantages of the prior art are solved by the features of claim 1. Preferred embodiments are defined in the dependent claims.

According to one aspect, there is provided a system for sanitizing a handle or railing comprises a retrofit covering for a handle or railing that includes a stability-enhancing interior element, and a cover of metal (preferably aluminum) that has a pathogen-eliminating outer surface. The system of the invention is particularly applicable to shopping cart handles but is useful for a wide variety of other handles and rails.

The stability-enhancing interior element comprises compression foam which operatively engages an existing handle or railing, such as by friction or by being attached thereto with an adhesive. Surrounding /jacketing the interior compression foam is a cover of a metal such as anodized aluminum with a pathogen-eliminating outer surface, which may comprise an antimicrobial or biocidal coating. Such coatings are described, for example, in US Patent Publ. 2005/0267233, PCT Publication WO/2021/113972, USP 6,929,705, or USP 8,900,716. The metal cover comprises two components which have interlocking or inter-engaging elements which allow the components to be mechanically connected together and held in place to provide a new, pathogen-eliminating surface.

According to another aspect, there is provided, in combination, an existing handle or railing having an outer surface; a compression foam structure, preferably adhesively secured to the outer surface of the handle; and a metal cover provided over the compression foam and having an outer surface, the outer surface having pathogen-eliminating properties. In one aspect, the metal cover is anodized aluminum with an antimicrobial or biocidal coating on the outer surface thereof. The cover comprises two components with interlocking or inter-engaging elements to hold them together. The existing handle or railing may comprise, for example, a shopping cart handle, although the present description would be applicable to other handles, railings, etc. Generally, the present description is applicable to any elongate structure.

According to yet another aspect, there is provided a method for retrofitting a sturdy sanitary cover on an existing handle or railing. The method comprises a) at least partially covering the existing handle or railing with a stability-enhancing element, and then b) jacketing the stability-enhancing element with a metal cover having an outer surface with pathogen-eliminating properties. In the method a) may be practiced by adhesively securing a compression foam to the existing handle or railing, and b) may be practiced utilizing an anodized aluminum cover with an outer coating of antimicrobial or biocidal material. In the method b) may be further practiced by interlocking together or inter-engaging two components.

The method is particularly applicable for use on a shopping cart handle although it could also be practiced on hospital bed railings, staircase railings, commercial building door handles, or other handles or rails.

In one aspect, the present description provides a robust system for sanitizing a handle or railing, and a method of retrofitting an existing handle or railing to provide an effective pathogen-eliminating user-engaging surface. This and other objects of the invention will become clear from a detailed description of the invention, and from the appended claims.

Thus, in one aspect, there is provided a covering for a handle or railing comprising: a stability-enhancing interior element adapted to be provided over the handle or railing; and a metal cover adapted to be provided over the stability-enhancing interior element; wherein the metal cover includes a pathogen-eliminating outer surface.

In another aspect, there is provided a system for sanitizing a handle or railing comprising a retrofit covering for the handle or railing; said retrofit covering including a stability-enhancing interior element, and a cover of metal that has a pathogen-eliminating outer surface.

In another aspect, there is provided a kit for providing a cover over a handle or railing, the kit comprising: a stability-enhancing interior element adapted to be provided over the handle or railing; and a metal cover adapted to be provided over the stability-enhancing interior element; wherein the metal cover includes a pathogen-eliminating outer surface.

In yet another aspect, there is provided a method for retrofitting a sanitary cover on an existing handle or railing, the method comprising:
a) at least partially covering the existing handle or railing with a stability-enhancing element; and
b) jacketing the stability-enhancing element with a metal cover having an outer surface with pathogen-eliminating properties.

### Brief Description of the Drawings

FIGURE 1 is a cross-sectional end view of a retrofit sanitary handle system covering an existing shopping cart handle;
FIGURE 2 is a side view of the handle system of FIGURE 1; and
FIGURE 3 is a perspective view of a shopping cart with the retrofit sanitary handle system of FIGURES 1 & 2 operatively provided thereon.

### Detailed Description

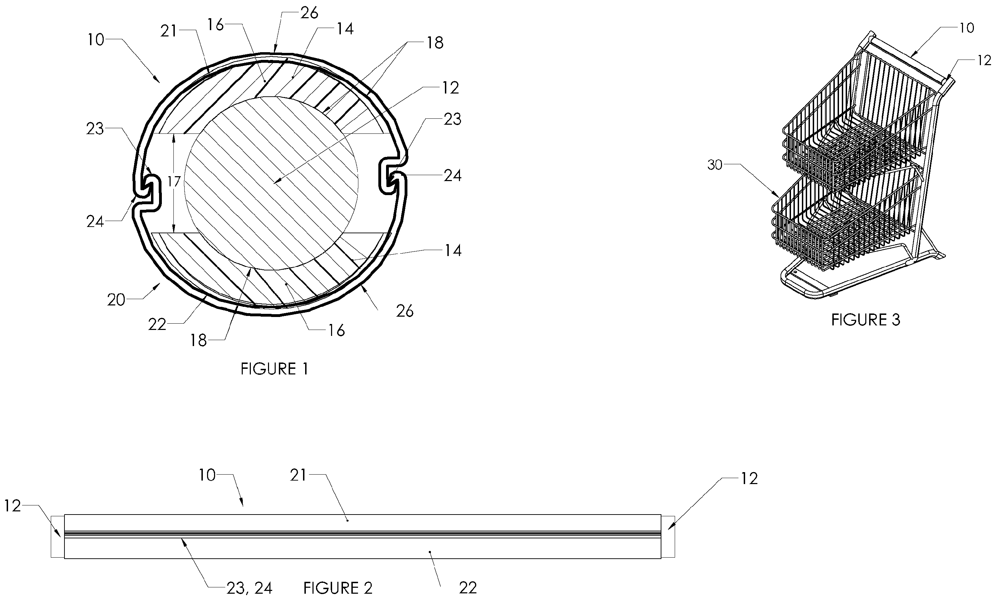

A retrofit sanitary handle system/covering according to the invention is shown generally by reference numeral 10 in FIGURES 1-3. The system 10 is adapted to be provided over an existing handle or railing 12, such as the handle of a shopping cart handle. It will be understood that the handle or railing 12 may comprise any such elongate article, such as a hospital bed railing, staircase railing, commercial building door handle, or a like handle or rail.

The system 10 includes a stability-enhancing interior element shown generally by reference numeral 14 in FIGURE 1. In the preferred embodiment the stability-enhancing element comprises compression foam 16 which may optionally be secured by adhesive 18 (shown exaggerated in size in FIGURE 1 for clarity of illustration only) to the handle or railing 12. In the embodiment illustrated, the foam 16 is provided in two portions which are spaced from each other as indicated by gaps 17 in FIGURE 1. In the illustrated embodiment, the two portions of the foam are provided as upper and lower portions for reasons discussed below. It will be understood that the sections may be provided on any other orientation over the handle 12.

In another aspect, the foam 16 may comprise a single sleeve that is wrapped or slid over the handle or railing 12. It will be appreciated that where the foam 16 is provided in two pieces, as shown in FIGURE 1 , the use of an adhesive to secure such pieces to the handle 12 facilitates installation. It will also be appreciated that where the foam 16 is a single sleeve, it may be possible to retain it in place over the handle 12 without the need for an adhesive.

The compression foam 16 preferably comprises a high or medium compression foam selected from the group consisting essentially of polyurethane, polyethylene, and neoprene foam, and combinations thereof. The foam may have a density of about 32- 641 Kg/m³ (2-40 lbs./ft.³), and which requires a pressure of about 0.28-1.03 bar (4-15 psi) to compress it 25%. Preferably the density is about 128- 481 Kg/m³ (8-30 lbs./ft.³ ) and requiring 0.41-0.83 bar (a psi of about 6-12) to compress it 25%. The adhesive 18 preferably comprises an acrylic and/or silicone adhesive with an effective operating range of -40°C to 49°C (-40 to 120°F). For providing the system of the present description on to a handle or railing 12, the surface of the handle or railing 12 is cleaned, such as with a liquid or spray isopropanol, or an alcohol wipe, and allowed to dry before applying adhesive 18. The foam 16 is then provided over the adhesive 18.

Jacketing the compression foam 16 and securely in engagement therewith is a metal cover shown generally by reference numeral 20 in FIGURE 1. The metal may be selected from aluminum (e. g. 6060-T5) such as anodized aluminum, titanium, stainless steel, or a variety of other metals, with anodized aluminum being preferred. In the embodiment illustrated in FIGURE 1 the cover 20 is formed by two components, an upper component 21, and a lower component 22. It will be understood from the present description that providing "upper" and "lower" components is preferred and that the components may be provided in other orientations as well. Mechanical elements are provided to connect the components 21, 22 by interlocking or inter-engaging such elements together. In the preferred embodiment as illustrated in FIGURE 1, the upper component 21 has one hook-shaped end 23 which receives an end projection 24 of the lower component 22, and vice-versa for the lower component 22. Alternatively, one component 21, 22 can have two hook-shaped ends 23 and the other component two end projections 24.

As can be seen in FIGURE 1, the mechanical elements allow engagement of opposed sides of each component 21 and 22. It is also noted that, in the preferred aspect, the upper component 22 is a mirror image of the lower component 21. In this way, one side (in FIGURE 1, the right side) of the upper component is adapted to fit under the adjacent side of the lower component, while the opposite side (in FIGURE 1, the left side) of the upper component is adapted to fit over the adjacent side of the lower component. This arrangement may be preferred as it allows one side of the cover 20 to be hooked together over the foam 16, to in essence form a hinge, after which the opposite sides can be brought together and snapped to engage the mechanical elements 23, 24. It will also be understood that the two components 21, 22, can be brought together simultaneously to form a snap fit along both sides.

The interlocking/inter-engaging ends 23, 24 are provided in the gaps 17 between the two portions of the foam 16, as seen in FIGURE 1 so that the components 21, 22 snap together, making a "clicking" sound when they do so. The ends 23, 24 securely fit together so that they will not separate during normal use of the handle system 10, by pressing the projections 24 into operative association with the hooks 23. The fit between the projections 24 and hooks 23 may be clearance, transition, or interference, preferably a transition or interference fit. In any case preferably the cover 20 is held securely in place, and securely engages the foam 16. Optionally another layer (not shown) of the same adhesive as 18 may be provided between the foam 16 and the interiors of components 21, 22.

As illustrated in FIGURE 1, when the two components 21 and 22 are connected, opposed seams are formed where the mechanical elements 23, 24 are joined. Aesthetically, it would be preferred for such seams to be provided on front and rear facing ends of a shopping cart handle, for instance. For this reason the components 21 and 22 would comprise "upper" and "lower" components so that the seams are not visible on a top view, where the palms of the user are typically placed.

As also illustrated in FIGURE 1 , the seams formed by the two components 21 and 22 are positioned in the vicinity of the gaps 17 if the foam 16 components are directly underneath respective components 21 , 22. Thus, where the components 21 and 22 are provided as "upper" and "lower" components, corresponding "upper" and "lower" foam 16 components would also be provided. The gaps 17 are preferred so as to avoid the foam material from interfering with the engagement of the mechanical elements 23, 24. However, as noted above, it would be possible to use a unitary foam piece.

As will be understood from the present description, as the two components 21 and 22 are connected the underlying foam 16 is compressed. In this state, the foam 16 exerts a radially outward force against the components 21 , 22, which serves to lock the components together owing the arrangement of the "hooks".

As an alternative to the components 21 , 22 which does not fall within the scope of the claimed invention, the cover 20 may be provided as a sleeve and crimped into contact with the foam substantially continuously along its length, or at one or more spaced locations.

The outer surface of the metal cover 20 has a pathogen-eliminating coating 26, shown greatly exaggerated in size in FIGURE 1 for clarity of illustration only. The coating 26 may be a known biocidal or antimicrobial coating such as provided by the UmanProtek^{™} technology of A3 Surfaces of Chicoutimi, QC, Canada, and/or provided by a variety of known or to be developed techniques, such as shown in US Patent Publ. 2005/0267233, PCT Publication WO/2021/113972, USP 6,929,705, and/or USP 8,900,716.

The covering 10 according to the description is shown in side view in FIGURE 2. The length thereof will, of course, depend upon the length of the handle or rail 12 and the covering 10 may terminate just short of the ends of the handle or rail 12, as schematically illustrated in FIGURE 2. An aspect of the description where the covering 10 is applied to the handle 12 of a conventional shopping cart 30 is illustrated in FIGURE 3.

The present description also provides a method for retrofitting a sturdy sanitary covering 10 on an existing handle or railing 12. The method comprises: a) at least partially covering the existing handle or railing 12 with a stability-enhancing element 16 (or 16/18) as shown in FIGURE 1; and then b) jacketing the stability-enhancing element 16 with a metal cover 20 having an outer surface (e. g. coating 26) with pathogen-eliminating properties. In the method a) may be practiced by adhesively (18) securing a compression foam 16 to the existing handle or railing 12. Also in the method b) may be practiced utilizing an anodized aluminum cover 20 with an outer coating 26 of antimicrobial or biocidal material. In the method b) may be further practiced by interlocking together or inter-engaging two components 21, 22 of the cover 20 (such as by using hook 23 and projection 24 ends thereof in a transition or interference fit, and snap together with a "clicking" sound) so that they are securely held together and preferably in secure contact with the stability-enhancing element 16; or alternatively b) is further practiced by tightly crimping a sleeve of aluminum into contact with the stability-enhancing element. In one aspect, a) and b) are practiced on a shopping cart handle 12 as the existing handle or railing, as seen in FIGURE 3 for shopping cart 30.

In the present description, the foam component 16 has been described as "stability-enhancing". As will be understood by persons skilled in the art, this term is intended to mean that the foam serves to securely hold the metal cover 20 in place once attached to a handle or rail 12. In particular, as described above, the foam 16 is adapted to compress when the metal cover components are secured together, whereby the radially outward force exerted by the foam retains the cover components 21, 22 together. Further, the outward force would preferably also be sufficient to frictionally engage the cover 20 over the foam 16 so as to prevent axial rotation there-between. To further enhance the latter, an adhesive may be used to secure the cover 20 to the foam 16. The compression of the foam 16 as described above will also exert a radially inward force against the handle or rail 12. This would serve to frictionally engage the foam 16 over the handle 12, thus preventing axial rotation of the foam, and therefore the cover 20, over the handle 12. As noted above, in a preferred aspect, the foam 16 may be affixed over the handle 12 using an adhesive. This would further serve to prevent axial rotation of the foam over the handle 12.

As noted above, the present description is directed to a handle cover that can be retrofit onto an existing handle, such as a rail or a handle on a shopping cart, etc. Accordingly, it will be understood that the handle cover, or covering, described herein may have any length or diameter that would be suitable for accommodating various sizes of rails, handles and the like. The description is therefore not limited to any particular size or relative size that may be depicted in the attached figures. The figures illustrate one particular aspect where the presently described cover has utility for protecting the handles of shopping carts.

Similarly, although the existing handle as shown at 12 in **FIGURE** 1 is illustrated as having a cylindrical outer surface, it will be appreciated that the present description is not limited to any specific shape of the handle. As will be understood, in view of the foam 16, the presently described handle cover may be fit, or retrofit, onto a wide variety of handle shapes.

**It** will also be appreciated that although the handle cover is shown as having a generally smoot, cylindrical outer surface, various other shapes may be possible. For example, the outer surface may have a ridged or textured surface or the cover may be axially curved.

In addition, given the retrofit nature of the description, it will be appreciated that the components of the present handle cover may be made commercially available in a kit form, whereby a purchaser would be able to secure the cover over a handle. In this regard, the kit may comprise the stability-enhancing, or foam, component 16, the cover component 20, and optionally, the respective adhesive(s) and/or instructions for assembling the handle cover over a handle. It will also be appreciated that the handle cover components may be provided in any length or lengths, which a user may cut to suit a given size requirement.

## Claims

1. A covering (10) for a handle or railing comprising:
a stability-enhancing interior element (16), in the form of a compression foam, adapted to be provided over the handle or railing; and
a metal cover (20) adapted to be provided over the stability-enhancing interior element (16);
wherein the metal cover (20) is provided with a pathogen-eliminating outer surface;
**characterized in that** said metal cover (20) comprises first and second cover components (21, 22), and wherein each of the components (21, 22) include respective mechanically interlocking elements (23, 24), whereby, when installed, the first and second components (21, 22) are adapted to surround at least a portion of the handle or railing and are securely held together and engage said compression foam.

2. The covering (10) of claim 1 further comprising an adhesive for affixing said stability-enhancing interior element (16) to the handle or railing and/or to the cover (20).

3. The covering (10) of claim 1 or 2, wherein said cover of metal (20) comprises an anodized aluminum cover with an antimicrobial or biocidal coating on the outer surface thereof.

4. A kit for providing a cover over a handle or railing, the kit comprising the covering (10) of claim 1.

5. The kit of claim 4 further comprising an adhesive for affixing said compression foam (16) to the handle or railing, when the covering (10) is installed.

6. The kit of claim 4 or 5, wherein said cover of metal (20) comprises an anodized aluminum cover with an antimicrobial or biocidal coating on the outer surface thereof.

7. The kit of any one of claims 4 to 6, wherein said metal cover (20) comprises first and second cover components (21, 22), and wherein each of the components (21, 22) include respective mechanically interlocking or inter-engaging elements (23, 24), whereby, when installed, the first and second components (21, 22) are securely held together and engage said stability enhancing interior element (16).

8. The covering (10) of any one of claims 1 to 3 or the kit of any one of claims 4 to 7, wherein the covering (10) is adapted to cover at least a portion of a handle of a shopping cart.

## Patentansprüche

1. Abdeckung (10) für einen Griff oder ein Geländer, umfassend:
ein stabilitätssteigerndes Innenelement (16) in Form eines Druckschaums, das über dem Griff oder dem Geländer bereitgestellt werden kann; und
eine Metallabdeckung (20), die über dem stabilitätssteigernden Innenelement (16) bereitgestellt werden kann;
wobei die Metallabdeckung (20) mit einer pathogeneliminierenden äußeren Oberfläche bereitgestellt ist;
**dadurch gekennzeichnet, dass** die Metallabdeckung (20) eine erste und eine zweite Abdeckungskomponente (21, 22) umfasst, wobei jede der Komponenten (21, 22) entsprechende mechanisch ineinander greifende Elemente (23, 24) einschließt, wodurch die erste und die zweite Komponente (21, 22), wenn sie installiert sind, geeignet sind, mindestens einen Abschnitt des Griffs oder des Geländers zu umgeben und sicher zusammengehalten werden und in den Druckschaum eingreifen.

2. Abdeckung (10) nach Anspruch 1, ferner umfassend einen Klebstoff zum Befestigen des stabilitätssteigernden Innenelements (16) am Griff oder Geländer und/oder an der Abdeckung (20).

3. Abdeckung (10) nach Anspruch 1 oder 2, wobei die Abdeckung aus Metall (20) eine anodisierte Aluminiumabdeckung mit einer antimikrobiellen oder bioziden Beschichtung auf ihrer äußeren Oberfläche umfasst.

4. Kit zum Bereitstellen einer Abdeckung über einem Griff oder einem Geländer, wobei das Kit die Abdeckung (10) nach Anspruch 1 umfasst.

5. Kit nach Anspruch 4, ferner umfassend einen Klebstoff zum Befestigen des Druckschaums (16) am Griff oder Geländer, wenn die Abdeckung (10) installiert ist.

6. Kit nach Anspruch 4 oder 5, wobei die Abdeckung aus Metall (20) eine anodisierte Aluminiumabdeckung mit einer antimikrobiellen oder bioziden Beschichtung auf ihrer äußeren Oberfläche umfasst.

7. Kit nach einem der Ansprüche 4 bis 6, wobei die Metallabdeckung (20) eine erste und eine zweite Abdeckungskomponente (21, 22) umfasst, und wobei jede der Komponenten (21, 22) entsprechende mechanisch ineinander greifende oder ineinander eingreifende Elemente (23, 24) einschließt, wodurch die erste und die zweite Komponente (21, 22), wenn sie installiert sind, sicher zusammengehalten werden und in das stabilitätsverbessernde Innenelement (16) eingreifen.

8. Abdeckung (10) nach einem der Ansprüche 1 bis 3 oder Kit nach einem der Ansprüche 4 bis 7, wobei die Abdeckung (10) geeignet ist, mindestens einen Abschnitt eines Griffs eines Einkaufswagens abzudecken.

## Revendications

1. Revêtement (10) pour une poignée ou une barre comprenant :
un élément intérieur améliorant la stabilité (16), sous la forme d'une mousse de compression, conçu pour être disposé sur la poignée ou la barre ; et
une enveloppe métallique (20) conçue pour être disposée sur l'élément intérieur améliorant la stabilité (16) ;
dans lequel l'enveloppe métallique (20) est dotée d'une surface extérieure éliminant les agents pathogènes ;
**caractérisé en ce que** ladite enveloppe métallique (20) comprend des premier et second composants d'enveloppe (21, 22), et dans lequel chacun des composants (21, 22) comporte des éléments d'emboîtement mécanique (23, 24) respectifs, moyennant quoi, une fois installés, les premier et second composants (21, 22) sont conçus pour entourer au moins une partie de la poignée ou de la barre et sont maintenus ensemble de manière sécurisée et viennent en prise avec ladite mousse de compression.

2. Revêtement (10) selon la revendication 1 comprend en outre un adhésif pour fixer ledit élément intérieur améliorant la stabilité (16) à la poignée ou à la barre et/ou à l'enveloppe (20).

3. Revêtement (10) selon la revendication 1 ou 2, dans lequel ladite enveloppe métallique (20) comprend une enveloppe en aluminium anodisé avec un traitement de surface antimicrobien ou biocide sur la surface extérieure de celle-ci.

4. Kit destiné à fournir une enveloppe sur une poignée ou une barre, le kit comprenant le revêtement (10) selon la revendication 1.

5. Kit selon la revendication 4 comprenant en outre un adhésif pour fixer ladite mousse de compression (16) à la poignée ou à la barre, lorsque le revêtement (10) est installé.

6. Kit selon la revendication 4 ou 5, dans lequel ladite enveloppe métallique (20) comprend une enveloppe en aluminium anodisé avec un traitement de surface antimicrobien ou biocide sur la surface extérieure de celle-ci.

7. Kit selon l'une quelconque des revendications 4 à 6, dans lequel ladite enveloppe métallique (20) comprend des premier et second composants d'enveloppe (21,22), et dans lequel chacun des composants (21,22) comporte des éléments d'emboîtement ou de mise en prise mécanique (23, 24) respectifs, moyennant quoi, lorsqu'ils sont installés, les premier et second composants (21,22) sont maintenus ensemble de manière sécurisée et viennent en prise dans ledit élément intérieur améliorant la stabilité (16).

8. Revêtement (10) selon l'une quelconque des revendications 1 à 3 ou kit selon l'une quelconque des revendications 4 à 7, dans lequel le revêtement (10) est conçu pour envelopper au moins une partie d'une poignée d'un chariot de courses.
